# EUROPÄISCHE PATENTSCHRIFT

(11) **EP 2 540 341 B1**
(45) Veröffentlichungstag und Bekanntmachung des Hinweises auf die Patenterteilung: **27.07.2016**
(21) Anmeldenummer: 12167278.6
(22) Anmeldetag: 09.05.2012
(51) Int. Cl.: A61N 1/362, A61N 1/05, A61N 1/36, A61N 1/39

(54) **Herzstimulator zur Abgabe einer kardialen Konktraktilitätsmodulationstherapie**
Cardiac stimulator for delivery of cardiac contractility modulation therapy
Stimulateur cardiaque destiné à administrer un traitement de modulation de la contractilité cardiaque

(30) Priorität: 28.06.2011 US 201161501768 P
(43) Veröffentlichungstag der Anmeldung: 02.01.2013
(73) Patentinhaber: BIOTRONIK SE & Co. KG, 12359 Berlin (DE)
(72) Erfinder: Dörr, Thomas, 12437 Berlin (DE)
(74) Vertreter: Galander, Marcus

(56) Entgegenhaltungen:
- WO-A1-2007/091244
- WO-A2-2007/029254

## Beschreibung

Die Erfindung betrifft einen Herzstimulator zur Abgabe einer kardialen Kontraktilitätsmodulationstherapie.

Implantierbare Herzstimulatoren in Form von Herzschrittmachern oder Cardiovertern/Defibrillatoren sind grundsätzlich bekannt. Solche Herzstimulatoren sind in der Regel an Elektrodenleitungen angeschlossen, die in einer Kammer eines Herzens oder in unmittelbarer Nähe Stimulations- und optional zusätzliche Defibrillationselektroden besitzen. Über eine Stimulationselektrode kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Eine derartige stimulierte Kontraktion einer Herzkammer wird im Rahmen dieser Beschreibung als stimuliertes Ereignis bezeichnet. Kommt es zu einer natürlichen Kontraktion der Herzkammer, wird dies im Rahmen dieser Beschreibung als Eigenaktion oder als natürliches oder intrinsisches Ereignis bezeichnet. Eine Kontraktion beispielsweise des rechten Atriums eines Herzens wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

Eine lokale Erregung des Myokards breitet sich vom Erregungsort ausgehend per Reizleitung im Myocard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myocards. Während der Phase der Depolarisation sind die Herzmuskelzellen für eine Erregung unempfindlich, d.h. refraktär. Diese Zeit wird als Refraktärzeit bezeichnet. Die mit der Depolarisation und Depolarisation einhergehenden elektrischen Potentiale können wahrgenommen und deren zeitlicher Verlauf - als Elektrokardiogramm bezeichnet - ausgewertet werden.

Beim gesunden Menschen wird der jeweilige Herzrhythmus durch den vom autonomen Nervensystem gesteuerten Sinusknoten bestimmt. Dieser erregt per Reizleitung das rechte Atrium eines menschlichen Herzens und weiter über den AV-Knoten den (rechten) Ventrikel des Herzens. Ein vom Sinusknoten ausgehender, natürlicher Herzrhythmus wird daher auch als Sinusrhythmus bezeichnet und führt zu jeweils natürlichen Kontraktionen der jeweiligen Herzkammer, die als natürliche (intrinsische) Ereignisse erfasst werden können.

Das Erfassen solcher natürlichen (intrinsischen) Ereignisse erfolgt durch Ableiten der elektrischen Potenziale des Myokards der jeweiligen Herzkammer mit Hilfe von Sensingelektroden, die Teil einer entsprechenden Elektrodenleitung sind. Dabei können die Sensingelektroden gleichzeitig die Stimulationselektroden sein und abwechselnd als Stimulations- und als Sensingelektroden verwendet werden. Typischerweise ist für das Sensing-d.h. die Wahrnehmung intrinsischer Ereignisse - ein Sensingelektrodenpaar vorgesehen, das von zwei benachbarten Elektroden, nämlich einer Spitzenelektrode (Tip-Elektrode) und einer Ringelektrode gebildet ist, von denen die Spitzenelektrode auch als Stimulationselektrode dient. Auf diese Weise erfolgt eine bipolare Ableitung eines intrakardialen Elektrokardiogramms (IEGM). Dabei erfolgt das Sensing und die Stimulation im Ventrikel mit Hilfe einer ventrikulären Elektrodenleitung und die Stimulation und das Sensing im Atrium (im rechten Atrium) mit einer atrialen Elektrodenleitung, die separat an den jeweiligen Herzstimulator angeschlossen sind. Zusätzlich kann auch eine linksventrikuläre Elektrodenleitung vorgesehen sein, die typischer Weise über den Coronarsinus und eine von diesem abzweigende Lateralvene in die Nähe des linken Ventrikels ragt und dort eine kleinflächige Stimulations- und/oder Sensingelektrode aufweisen kann.

Die Sensingelektroden sind im Betrieb des Herzstimulators mit entsprechenden Sensingeinheiten verbunden, die ausgebildet sind, ein jeweiliges über eine Sensingelektrode (bzw. ein Sensingelektrodenpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch Schwellwertverglcich, d.h. ein intrinsisches Ereignis wird detektiert, wenn ein jeweiliges intrakardiales Elektrokardiogramm einen geeignet vorgegebenen Schwellwert überschreitet.

Aus der Frequenz, mit der atriale bzw. ventrikuläre Ereignisse aufeinander folgen, kann die jeweilige intrinsische atriale Herzrate (atriale Frequenz) bzw. ventrikuläre Herzrate (Ventrikelfrequenz) abgeleitet und somit beispielsweise Tachykardien detektiert werden.

Das Erfassen natürlicher Ereignisse dient bei bekannten Demand-Schrittmachern außerdem der Unterdrückung (Inhibierung) der Abgabe von Stimulationsimpulsen an eine entsprechende Herzkammer, falls das natürliche Ereignis in einem Zeitfenster vor der geplanten Abgabe eines Stimulationsimpulses an diese Herzkammer erfasst wird. Bei ratenadaptiven Herzschrittmachern wird der Zeitpunkt der Abgabe eines jeweiligen Stimulationsimpulses in Abhängigkeit einer jeweiligen Stimulationsrate geplant, die dem physiologischen Bedarf eines Patienten entsprechen soll, also bei größerer Anstrengung beispielsweise höher ist. Hierzu kann ein Herzstimulator mit einem oder mehreren Aktivitätssensoren ausgestattet sein, der beispielsweise ein nachfolgend näher beschriebener CLS-Sensor sein kann.

Die natürliche Einwirkung des autonomen Nervensystems auf die Herzrate (Herzfrequenz), die durch einen ratenadaptiven Herzstimulator nachgebildet werden, wird als Chronotropie bezeichnet.

Neben der Chronotropie wird die Herzleistung auch von dessen Kontraktilität bestimmt, deren Beeinflussung als Inotropie bezeichnet wird.

Zur Bestimmung der Kontraktilität eines Herzen ist es bekannt, in einem Gehäuse eines Herzstimulators (z.B. eines implantierbaren Herzschrittmachers) eine Impedanz- oder Leitfähigkeitsmesseinheit anzuordnen, die ausgebildet ist, ein uni- oder bipolares Impedanzoder Leitfähigkeitsverlaufssignal zu erzeugen. Dazu werden während wenigstens eines Herzzyklus' mehrere Impedanz- oder Leitfähigkeitswerte oder ein entsprechender Impedanz- oder Leitfähigkeitsverlauf gemessen. Dies geschieht entweder unipolar durch Messen zwischen einer Neutralelektrode und einer Messelektrode oder zwischen zwei Messelektroden. Außerdem ist in dem Gehäuse eine Auswerteeinheit angeordnet, die zum Auswerten des Impedanz- oder Leitfähigkeitsverlaufs und zum Ableiten eines Kontraktilitätswertes aus dem Impedanz- oder Leitfähigkeitsverlauf dient. Elektrotherapiegeräte, die die Kontraktilität eines Herzens ermitteln können, bieten die Möglichkeit, einer von dem Elektrotherapiegerät abzugebende Therapie an den jeweiligen Kontraktilitätszustand des Herzens des Patienten anzupassen.

Wie angedeutet, beschreibt die Kontraktilität einen inotropen Zustand eines Herzens. Die Kontraktilität beeinflusst die Kraft und die Geschwindigkeit einer myokardialen Kontraktion. Die Kontraktilität wird durch drei Mechanismen gesteuert:
eine direkte Steuerung durch das autonome Nervensystem (ANS),
den sogenannten Starling-Mechanismus, und
den sogenannten Bowditch-Effekt (Kraft-Frequenzkopplung).

Der Hauptmechanismus, die Steuerung der Kreislaufregulation durch das autonome Nervensystem, vergrößert die Kontraktilität und die Herzrate, wenn ein erhöhter metabolischer Bedarf vorliegt, beispielsweise bei körperlicher oder physischer Anstrengung, um eine geeignete Blutversorgung sicherzustellen. Beim gesunden Menschen bewirkt die Inotropie des Herzens somit einen Anstieg der Kontraktilität bei erhöhten physiologischen Bedarf.

Bei Patienten mit chronischem Herzversagen (Chronic Heart Failure, HF) nimmt die myokardiale Kontraktilität bis auf ein niedriges Niveau ab und die interventrikuläre Synchronisation wird verschlechtert. Dies wird von einem geringen Auswurfanteil (Ejection Fraction, EF) begleitet, sowie von einer geringen Lebensqualität und einer hohen Sterblichkeit. HF kommt in der Bevölkerung häufig vor. In jüngerer Zeit werden HF-Patienten mit Resynchronisationstherapie-Geräten, beispielsweise 3-Kammer-Herzschrittmachern oder Defibrillatoren behandelt. Ziel einer solchen Schrittmachertherapie ist es, die zwei Ventrikel eines Herzens durch biventrikuläre Stimulation zu synchronisieren, um das Zeitverhalten der Herzkammern zu verbessern und damit die Herzleistung. Eine derartige Therapie wird auch als kardiale Resynchronisationstherapie (Cardiac Resynchronisation Therapy, CRT) bezeichnet. Die kardiale Resynchronisationstherapie (CRT) ist hinreichend bekannt und wird z.B. von den Biotronik CRT-D Implantaten (Lumax HF-T) angeboten.

Da die Kontraktilität des Herzens physiologisch vom autonomen Nervensystem gesteuert wird, kann die Erfassung der Kontraktilität auch zum Einstellen einer physiologisch adäquaten Stimulationsrate bei ratenadaptiven Herzschrittmachern genutzt werden. Diese weiter vorne bereits angesprochene Art der Stimulationsratensteuerung ist auch als Closed Loop Stimulation (CLS) bekannt.

Für die CLS wird der intrakardiale Impedanzverlauf nach Beginn der Ventrikelkontraktion gemessen. Diese Messung erfolgt sowohl bei intrinsischen als auch bei stimulierten Ereignissen. Es besteht eine direkte Abhängigkeit zwischen dem rechtsventrikulären Impedanzverlauf und der Kontraktionsdynamik. Die Kontraktionsdynamik wiederum ist ein Parameter für die Stimulation des Herzens durch den Sympathikus.

Die Closed Loop Stimulation dient wie gesagt der Stimulationsratensteuerung bei einem ratenadaptiven Herzschrittmacher.

Zur Steigerung der Kontraktilität einer Herzkammer ist die sogenannte kardiale Kontraktilitätsmodulationstherapie (Cardiac Contractility Modulation: CCM) bekannt.

Hierfür bietet beispielsweise die Firma Impulse Dynamics ein sogenanntes OPTIMIZER-System zur CCM-Therapie an. Dieses System umfasst einen Stimulationsimpulsgenerator, verbunden mit 3 Elektroden, von denen im Betrieb eine im Atrium und 2 am Septum des rechten Ventrikels eines Patienten angeordnet sind. Das Therapieprinzip beruht auf einer Abgabe von biphasischen Stimulationspulsen mit einer Amplitude von 7-10V und einer Gesamtimpulsdauer von ∼20ms in die absolute Refraktärzeit des Ventrikels mit dem Ziel, die Kontraktilität zu steigern. Die Therapie wird dabei für bestimmte Zeiteinheiten des Tages (z.B. abwechselnd jeweils 1h ein, 1h aus) abgegeben.

Das Prinzip der kardialen Kontraktilitätsmodulationstherapie ist unter anderem auch in US 6,317,631 B1 beschrieben.

Die Wirkung der CCM-Therapie beruht - so wird derzeit vermutet - in einer Modifikation des zellulären Kalziumionenaustauschs und führt so zu einer gesteigerten Kontraktionskraft, die auch bei einem vorliegenden Vorhofflimmern einen therapeutischen Benefit zur Folge haben sollte. Dies ist zwar klinisch noch nicht nachgewiesen, ist pathophysiologisch aber nachvollziehbar.

US 2010/0069977 A1, US 2010/0069980 A1, US 2010/0069984 A1, US 2010/0069985 A1 beschreiben Methoden, um eine CCM-Stimulation bedarfsgerecht abzugeben. Hier wird der Einsatz von physiologischen Sensoren, Nieren- oder Herzfunktionssensoren, Elektrolytsensoren, Serumsensoren (z.B. Kreatinin), Neurosensoren (Vagus, Sympathikus), Adverse Event Detektoren, Worsening Heart Failure Sensoren, MRT-Sensoren, Aktivitätssen-soren, Schlafapnoe-Sensoren, Ischemiesensoren, Sensoren für den methabolischen Bedarf und Infarktsensoren sowie Herzrhythmus-abhängige CCM-Steuerungen allgemein beschrieben. In dem genannten Stand der Technik wird auch eine Abschaltung der CCM-Therapie bei erkanntem Vorhofflimmern bzw. Vorhofarrhythmien (siehe US 2010/0069977 Fig. 20A und [0332]) beschrieben. Ebenso wird dort ganz allgemein auf die mögliche Kombination von CCM mit anderen Stimulations- und Elektrotherapieformen, wie CRT, ICD und Neurostimulation eingegangen.

Ein Gerät nach der Präambel von Anspruch 1 ist von WO 2007/029254 bekannt. Der Erfindung liegt die Aufgabe zugrunde, einen verbesserten Herzstimulator zu schaffen. Erfindungsgemäß wird diese Aufgabe durch einen Herzstimulator gelöst. Dieser umfasst
- wenigstens eine Stimulationseinheit, die mit einer oder mehreren Stimulationselektroden verbunden oder zu verbinden ist und ausgebildet ist, auf ein entsprechendes Ansteuersignal hin Stimulationsimpulse zur Abgabe über wenigstens eine Stimulationselektrode zu generieren, sowie
- mit einer Herzrhythmuserfassungseinheit, die ausgebildet ist, wenigstens einen atrialen Herzrhythmus zu erfassen und
- eine Steuereinheit, die mit der Stimulationseinheit und der Herzrhythmuserfassungseinheit verbunden und ausgebildet ist, eine Stimulationsimpulsabgabe über die Stimulationseinheit derart zu steuern, dass der Herzstimulator überschwellige Stimulationsimpulse zur Anregung einer Kontraktion einer Herzkammer und unterschwellige Stimulationsimpulse für eine kardiale Kontraktionsmodulationstherapie abgeben kann.

Erfindungsgemäß ist die Steuereinheit ausgebildet, in Reaktion auf ein Erfassen einer atrialen Arrhythmie durch die Herzrhythmuserfassungseinheit die Stimulationseinheit derart anzusteuern, dass diese unterschwellige Stimulationsimpulse für eine kardiale Kontraktili-tätsmodulationstherapie im Rahmen einer kardialen Kontraktilitätsmodulationstherapie erzeugt und abgibt und in Reaktion auf ein Erfassen eines Sinusrhythmus durch die Herzrhythmuserfassungseinheit die Stimulationseinheit derart anzusteuern, dass diese wenigstens bei Bedarf überschwellige Stimulationsimpulse zur Anregung einer Kontraktion einer Herzkammer abgeben kann, aber keine unterschwelligen Stimulationsimpulse für eine kardiale Kontraktionsmodulationstherapie.

Mit anderen Worten wird die Aufgabe durch einen kombinierten Herzstimulator zur CRT-Stimulation und CCM-Stimulation gelöst, der mit einer Rhythmusbewertungseinheit verbunden ist, die entweder einen vorhandenen Sinusrhythmus nachweisen oder eine atriale Arrhythmie klassifizieren kann und der eine zusätzliche Therapieauswahleinheit aufweist, wobei die Therapieauswahleinheit die Abgabe entweder der CRT-Therapie oder der CCM-Therapie anhand der Klassifikation des Vorhofrhythmus derart auswählt, dass eine CRT-Therapie bei Sinusrhythmus bevorzugt wird und die CCM-Therapie bei Vorhofarrhythmie abgegeben wird.

Dieser Herzstimulator ist somit eine Vorrichtung zum kombinierten Einsatz von kardialer Resynchronisationstherapie (CRT) und kardialer Kontraktilitätsmodulation (CCM) in einem elektronischen Implantat.

Der Herzstimulator ist in der Lage, die jeweilige Therapieform so auszuwählen, dass jeweils diejenige Therapie aktiviert wird, die im Augenblick den größten Therapiebenefit für den Patienten darstellt. Auf diese Weise ist der Herzstimulator in der Lage, die kardiale Kontraktilitätsmodulationstherapie (Cardiac Contractility Modulation: CCM) und die kardiale Resynchronisationstherapie (CRT) derart zu kombinieren, dass ein maximaler zu erwartender klinischer Nutzen für den Patienten entsteht, indem die jeweilige Therapie immer dann eingesetzt wird, wenn diese das überlegene Behandlungskonzept darstellt.

Der erfindungsgemäße Herzstimulator bietet den Vorteil, dass abhängig vom Rhythmuszu-stand eines Herzinsuffizientpatienten die jeweils wirksamste Therapie automatisch ausgewählt wird und so eine Therapieeffizienzsteigerung erzielt wird. Die Vorteile der CCM-Therapie und der CRT werden so in einem Gerät kombiniert.

Die Erfindung schließt die Erkenntnis ein, dass US 2010/0069977 A1, US 2010/0069980 A1, US 2010/0069984 A1, US 2010/0069985 A1 zwar eine Abschaltung der CCM-Therapie bei einem erkannten Vorhofflimmern bzw. Vorhofarrhythmien beschreiben. Allerdings wird hier nicht auf den Grund der CCM-Abschaltung eingegangen. Man kann vermuten, dass die CCM-Therapie bei Vorhofarrhythmien ggf. nicht mehr korrekt synchronisiert werden kann. Allerdings ist dies bei einem an das ventrikuläre Ereignis anknüpfende CCM-Timing nicht nachvollziehbar.

Da die CCM-Therapie jedoch gerade als Therapieoption für sog. CRT-Non-Responder bevorzugt wird und Vorhofarrythmie eine der bekannten Ursachen für eine CRT-Non-Response ist, erkennt die Erfindung hier einen erfolgversprechenden Ansatz, die CCM-Therapie gerade beim Vorliegen von Vorhofarrhythmien zu aktivieren.

US 2010/0069977 A1, US 2010/0069980 A1, US 2010/0069984 A1, US 2010/0069985 A1 beschreiben zwar auch die mögliche Kombination von CCM und CRT-Stimulation in einem Gerät, gehen aber in keiner spezifischen Weise auf die möglichen Umschaltungen zwischen CCM und CRT-Stimulation ein. Es wird lediglich eine unterschiedliche Stimulationsschwelle von CRT-Stimulation und CCM betrachtet.

Der hier vorgestellte Herzstimulator beruht auf der Annahme, dass bei Sinusrhythmus die CRT-Stimulation einer CCM-Therapie hinsichtlich des therapeutischen Benefits deutlich überlegen ist. Bei Vorhoffarrhythmien, insbesondere Vorhofflimmern hingegen ist (zumindest bei ausgewählten Patienten) die CCM-Therapie jedoch wirksamer als die CRT-Stimulation. Hintergrund ist, dass die CRT-Stimulation eine mechanische Resynchronisation des Herzens bewirkt, wobei der Benefit nur dann bewiesen wurde, wenn neben der interventrikulären Resynchronisation eine adäquate atrio-ventrikuläre Synchronisation vorliegt. Andernfalls wird der positive Effekt der CRT-Stimulation durch die fehlende AV-Synchronisation mehr als egalisiert.

Vorzugsweise umfasst die Herzrhythmuserfassungseinheit eine atriale Rhythmusbewer-tungseinheit, die mit einer zum Platzieren im rechten Atrium eines Herzens konfigurierten atrialen Elektrode verbunden ist.

Vorzugsweise ist die Herzrhythmuserfassungseinheit und ggf. deren atriale Rhythmusbewertungseinheit ausgebildet, die Frequenz und Stabilität eines ventrikulären Rhythmus auszuwerten, um ein Vorhofflimmern zu diagnostizieren.

Vorzugsweise ist der Herzstimulator ein implantierbarer Kardioverter/Defibrillator (ICD) und/oder ein biventrikulärer Herzschrittmacher (CRT-D) und/oder ein Neurostimulator.

Vorzugsweise ist die Steuereinheit so konfiguriert, dass die kardiale Kontraktilitätsmodulationstherapie (CCM-Therapie) zusätzlich durch einen weiteren Sensor gesteuert (Aktivität, Methabolischer Bedarf, Closed Loop Stimulation (CLS), ventrikuläres Potential, Nierenfunktionssensor, Lungenfunktionssensor, Herzfunktionssensor, Elektrolytsensor, Neurosensor/Vagus/Sympathikus, Adverse Event Sensor, Worsening Heart Failure Sensor, Sleep apnea Sensor, Ischemiesensor, Infarktsensor, etc.).

Vorzugsweise ist der Herzstimulator gleichzeitig auch ein implantierbarer Kardioverter zur elektrischen Konversion von Vorhofflimmern, wobei die Steuereinheit so konfiguriert ist, dass einem Kardioversionsversuch des Vorhofflimmerns immer eine definierte Phase einer CCM-Therapie zur Verbesserung des Kardioversionserfolges vorangeht. Ein derartiger bevorzugter Herzstimulator dient dem Ziel, durch die CCM-Therapie die linksatrialen Druckverhältnisse zunächst zu normalisieren, bevor eine Kardioversion stattfindet.

Zur Lösung der Aufgabe wird auch ein Verfahren zur Therapie eines Herzens vorgeschlagen, bei dem entweder eine kardiale Kontraktilitätsmodulationstherapie (CCM) oder eine kardiale Resynchronisationstherapie (CRT) abgegeben wird und die kardiale Kontraktilitätsmodulationstherapie dann abgegeben wird, wenn eine atriale Arrhythmie vorliegt und die kardiale Resynchronisationstherapie dann abgegeben wird, wenn keine atriale Arrhythmie vorliegt.

Eine Variante des Verfahrens betrifft ein Betriebsverfahren für einen entsprechenden Herzstimulator auch zu nicht-therapeutischen Zwecken.

Die Erfindung soll nun anhand von Ausführungsbeispielen mit Bezug auf die Abbildungen näher erläutert werden. Von diesen zeigt:
- Fig. 1: einen Herzstimulator als CRT-CCM-Stimulator;
- Fig. 2: ein Blockschaltbild wesentlicher Komponenten des Herzstimulators; und
- Fig. 3: ein Ablaufdiagramm für die Therapieauswahl.

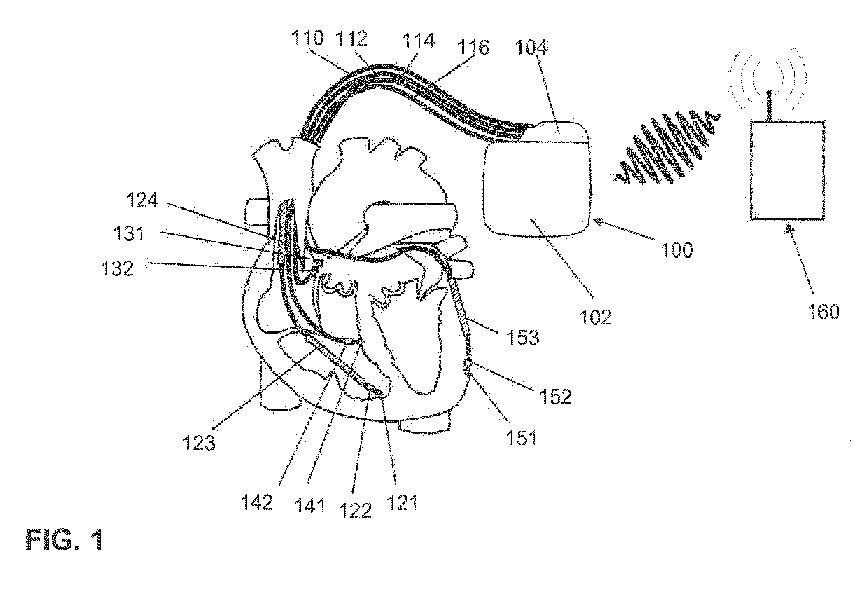

In der Fig. 1 ist als Implementierungsbeispiel ein Dreikammer-ICD-System mit umschaltbarer CRT-CCM-Funktion dargestellt. Der Herzstimulator (Impulsgenerator oder auch schlicht Generator) 100 ist mit mehreren implantierbaren Elektrodenleitungen 110, 112, 114 und 116 verbunden. Der Generator 100 besitzt ein Gehäuse 102, das im Beispielsfall aus Metall und daher elektrisch leitend sowie hermetisch dicht ist. In dem Gehäuse 102 sind elektronische und elektrische Komponenten des Generators 100 wie z.B. eine Batterie, Steuerelektronik, Stimulationsimpulsgeneratoren, Wahrnehmungseinheiten (auch als Sensing-Einheiten bezeichnet) mit entsprechender Verstärker- und Filterelektronik untergebracht. Diese Komponenten sind über elektrische Steckverbindungen in einem Anschlussgehäuse 104 (auch als Header bezeichnet) des Generators 100 mit den Elektrodenleitungen 110, 112, 114 und 116 lösbar verbunden.

Zur rechtsventrikulären Wahrnehmung und Stimulation umfasst die rechtsventrikuläre Elektrodenleitung 110 eine rechtsventrikuläre Tipelektrode 121 und eine rechtsventrikuläre Ringelektrode 122 am distalen Ende. Über die rechtsventrikuläre Tipelektrode 121 werden die rechtsventrikulären Stimulationsimpulse für die biventrikuläre CRT-Stimulation abgegeben. Zur Defibrillationsschockabgabe sind an dieser rechtsventrikulären Elektrodenleitung 110 eine distale Schockwendel 123 und optional auch eine proximale Schockwendel 124 angebracht. Die Gegenelektrode bildet hier das Generatorgehäuse 100.

Die rechtsatriale Elektrodenleitung 116 weist an ihrem distalen Ende einen bipolaren Wahrnehmungs- und Stimulationspol (mit einer rechtsatrialen Tip-Elektrode 131 und einer rechtsatrialen Ring-Elektrode 132) auf und dient der Wahrnehmung des atrialen Rhythmus und bei Bedarf der atrialen Stimulation.

Zur CCM-Stimulation - also zur kardialen Kontraktilitätsmodulationstherapie - sind ein oder mehrere rechtsventrikuläre Septale-Elektrodenleitungen 112 vorgesehen, die über eine Tipelektrode 141 und Ringelektrode 142 die CCM-Pulse jeweils an das ventrikuläre Septum abgeben.

Außerdem umfasst das System auch eine linksventrikuläre bzw. CS-(Coronar Sinus) Elektrodenleitung 114 zur Angabe von linksventrikulären Stimulationsimpulsen zur CRT über eine linksventrikuläre Tipelektrode 151 und eine linksventrikuläre Ringelektrode 152. Zur effektiveren Defibrillation/Kardioversion ist optional eine linksventrikuläre Schockwendel 153 möglich.

Die Kommunikation mit externen Programmier- und Steuer- und Datenübertragungsgeräten 160 erfolgt über eine drahtlose, bidirektionale Telemetrieeinheit.

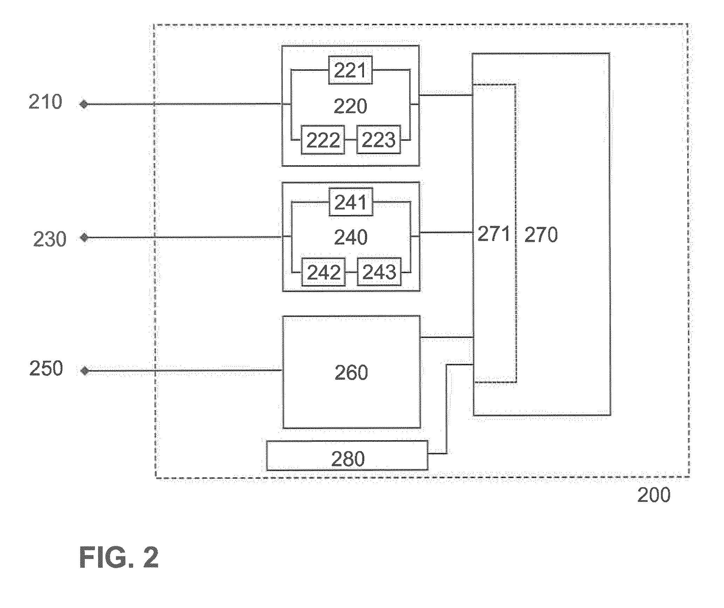

In der Fig. 2 ist ein schematisches Blockschaltbild eines kombinierten CRT-CCMStimulators dargestellt. Dieser umfasst mindestens folgende Komponenten: Generator 200; rechtsatriale-Elektrode 210, verbunden mit einer atrialen Wahrnehmungs- und Stimulationseinheit 220 zur Klassifikation des atrialen Rhythmus und bedarfsgesteuerten atrialen Stimulation; rechts- und linksventrikuläre Elektrodenanschlüsse 230, verbunden mit einer biventrikulären Wahrnehmungs- und Stimulationseinheit 240 zur Wahrnehmung und Klassifikation des ventrikulären Rhythmus und der biventrikulären CRT-Stimulation sowie einer oder mehrerer CCM-Elektroden 250, verbunden mit einer CCM-Stimulationseinheit 260, wobei die atriale Wahrnehmungs- und Stimulationseinheit 220, die biventrikuläre Wahrnehmungs- und Stimulationseinheit 240 und die CCM-Stimulationseinheit 260 mit einer Steuereinheit 270 verbunden sind. Die Steuereinheit 270 enthält eine Therapieauswahleinheit 271. Diese Therapieauswahleinheit 271 aktiviert entsprechend des klassifizierten atrialen Rhythmus entweder die biventrikuläre Stimulationseinheit 240 oder die CCM-Stimulationseinheit 260.

Die biventrikuläre Wahrnehmungs- und Stimulationseinheit 240 umfasst eine an sich bekannte ventrikuläre Stimulationseinheit 241 und Sensingeinheit 242. Außerdem enthält die biventrikuläre Wahrnehmungs- und Stimulationseinheit 240 eine ventrikuläre Rhythmusbewertungseinheit 243.

Die atriale Wahrnehmungs- und Stimulationseinheit 220 ist zur Klassifikation des atrialen Rhythmus und bedarfsgesteuerten atrialen Stimulation des Atriums ausgebildet und umfasst die an sich bekannte atriale Stimulationseinheit 221 und Sensingeinheit 222. Außerdem enthält die atriale Wahrnehmungs- und Stimulationseinheit 220 eine atriale Rhythmusbewertungseinheit 223, die eine Klassifikation des atrialen Rhythmus in an sich bekannter Weise durchführt und z.B. durch Analyse der atrialen Herzrate in der Lage ist, atriale Fibrillationen zu erkennen und von nicht pathologischen atrialen Rhythmen zu unterscheiden. Das Ausgangssignal der atrialen Rhythmusbewertungseinheit 223 wird der Therapieauswahleinheit 271 zugeführt.

Ein hämodynamischer oder Aktivitätssensor 280 liefert im Betrieb ein Ausgangssignal, welches den metabolischen Bedarf eines Patienten widerspiegelt und welches von der Therapieauswahleinheit 271 ebenfalls ausgewertet werden kann.

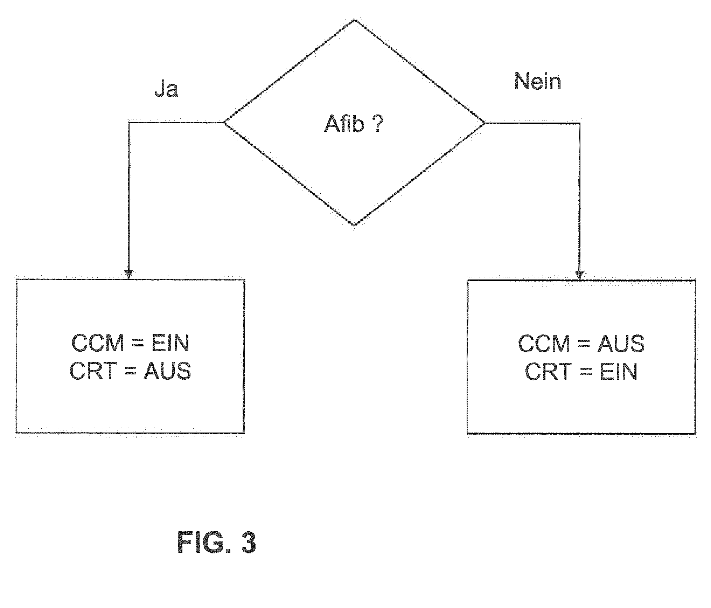

Fig. 3 zeigt die Funktionsweise der Therapieauswahleinheit 271 anhand eines Ablaufdiagramms für die Therapieauswahl. Erkennt die atriale Rhythmusbewertungseinheit 223 ein Vorhofflimmern bzw. eine Vorhofarrhythmie (Afib), schaltet die Therapieauswahleinheit 271 die CCM-Stimulation ein und die biventrikuläre Stimulation aus. Liegt kein Vorhofflimmern vor, schaltet die Therapieauswahleinheit 271 die CCM-Stimulation ab und die biventrikuläre Stimulation ein.

## Patentansprüche

1. Herzstimulator
- mit wenigstens einer Stimulationseinheit, die mit einer oder mehreren Stimulationselektroden verbunden oder zu verbinden ist und ausgebildet ist, auf ein entsprechendes Ansteuersignal hin Stimulationsimpulse zur Abgabe über wenigstens eine Stimulationselektrode zu generieren,
- mit einer Herzrhythmuserfassungseinheit, die ausgebildet ist, wenigstens einen atrialen Herzrhythmus zu erfassen und
- mit einer Steuereinheit, die mit der Stimulationseinheit und der Herzrhythmuserfassungseinheit verbunden und ausgebildet ist, eine Stimulationsimpulsabgabe über die Stimulationseinheit derart zu steuern, dass der Herzstimulator überschwellige Stimulationsimpulse zur Anregung einer Kontraktion einer Herzkammer und unterschwellige Stimulationsimpulse für eine kardiale Kontraktionsmodulationstherapie abgeben kann,
wobei die Steuereinheit ausgebildet ist, in Reaktion auf ein Erfassen einer atrialen Arrhythmie durch die Herzrhythmuserfassungseinheit die Stimulationseinheit derart anzusteuern, dass diese unterschwellige Stimulationsimpulse für eine kardiale Kontraktionsmodulationstherapie abgeben kann,
**dadurch gekennzeichnet,**
**dass** die Steuereinheit weiters ausgebildet ist in Reaktion auf ein Erfassen eines Sinusrhythmus durch die Herzrhythmuserfassungseinheit die Stimulationseinheit derart anzusteuern, dass diese überschwellige Stimulationsimpulse zur Anregung einer Kontraktion einer Herzkammer abgeben kann, aber keine unterschwelligen Stimulationsimpulse für eine kardiale Kontraktionsmodulationstherapie.

2. Herzstimulator nach Anspruch 1, **dadurch gekennzeichnet, dass** die Herzrhythmuserfassungseinheit eine atriale Rhythmusbewertungseinheit umfasst, die mit einer zum Platzieren im rechten Atrium eines Herzens konfigurierten atrialen Elektrode verbunden oder zu verbinden ist.

3. Herzstimulator nach Anspruch 1 oder 2, **dadurch gekennzeichnet, dass** die Herzrhythmuserfassungseinheit und ggf. eine dieser zugeordnete atriale Rhythmusbewertungseinheit ausgebildet ist, die Frequenz und Stabilität eines ventrikulären Rhythmus auszuwerten, um ein Vorhofflimmern zu diagnostizieren.

4. Herzstimulator nach einem der Ansprüche 1 bis 3, **dadurch gekennzeichnet, dass** der Herzstimulator ein implantierbarer Kardioverter/Defibrillator (ICD) und/oder ein biventrikulärer Herzschrittmacher (CRT-D) und/oder ein Neurostimulator ist.

5. Herzstimulator nach Anspruch 4, **dadurch gekennzeichnet, dass** der Herzstimulator gleichzeitig auch ein implantierbarer Kardioverter zur elektrischen Konversion von Vorhofflimmern ist, wobei die Steuereinheit so konfiguriert ist, dass einem Kardioversionsversuch des Vorhofflimmerns immer eine definierte Phase einer CCM-Therapie zur Verbesserung des Kardioversionserfolges vorangeht.

6. Herzstimulator nach einem der Ansprüche 1 bis 3, **dadurch gekennzeichnet, dass** der Herzstimulator zusätzlich einen weiteren Sensor aufweist, der ein die Aktivität, den metabolischen Bedarf und/oder das ventrikuläre Potential widerspiegelnde Ausgangssignal liefert oder ein Nierenfunktionssensor, Lungenfunktionssensor, Herzfunktionssensor, Elektrolytsensor, Neurosensor/Vagus/Sympathikus, Adverse Event Sensor, Worsening Heart Failure Sensor, Sleep apnea Sensor, Ischemiesensor oder Infarktsensor ist und der mit der Steuereinheit verbunden ist, wobei die Steuereinheit ausgebildet ist, das Ausgangssignal dieses Sensors auszuwerten und zur Steuerung der Kontraktilitätsmodulation zu nutzen.

## Claims

1. A cardiac stimulator
- comprising at least one stimulation unit, which is connected or connectable to one or more stimulation electrodes and which is configured to generate stimulation pulses to be delivered via at least one stimulation electrode in response to a relevant trigger signal,
- comprising a cardiac rhythm detection unit, which is configured to detect at least one atrial cardiac rhythm, and
- comprising a control unit, which is connected to the stimulation unit and the cardiac rhythm detection unit and which is configured to control a delivery of a stimulation pulse via the stimulation unit such that the cardiac stimulator can deliver above-threshold stimulation pulses to stimulate contraction of a ventricle, and sub-threshold stimulation pulses for cardiac contraction modulation therapy,
wherein
the control unit is configured tocontrol the stimulation unit in response to detection of atrial arrhythmia by the cardiac rhythm detection unit, such that said stimulation unit can deliver sub-threshold stimulation pulses for cardiac contraction modulation therapy,
**characterised in that**
the control unit is also configured to control the stimulation unit in response to detection of a sinus rhythm by the cardiac rhythm detection unit, such that said stimulation unit can deliver above-threshold stimulation pulses to stimulate a contraction of a ventricle, but not sub-threshold pulses for cardiac contraction modulation therapy.

2. The cardiac stimulator according to claim 1, **characterised in that** the cardiac rhythm detection unit comprises an atrial rhythm evaluation unit, which is connected or connectable to an atrial electrode configured for placement in the right atrium of a heart.

3. The cardiac stimulator according to claim 1 or 2, **characterised in that** the cardiac rhythm detection unit and, where applicable, an atrial rhythm evaluation unit assigned thereto is/are configured to evaluate the frequency and stability of a ventricular rhythm in order to diagnose atrial fibrillation.

4. The cardiac stimulator according to any one of claims 1 to 3, **characterised in that** the cardiac stimulator is an implantable cardioverter/defibrillator (ICD) and/or a biventricular cardiac pacemaker (CRT-D) and/or a neurostimulator.

5. The cardiac stimulator according to claim 4, **characterised in that** the cardiac stimulator is also an implantable cardioverter for electrical conversion of atrial fibrillation, wherein the control unit is configured such that a defined phase of CCM therapy always precedes a cardioversion attempt in response to atrial fibrillation in order to improve a success of cardioversion.

6. The cardiac stimulator according to any one of claims 1 to 3, **characterised in that** the cardiac stimulator additionally comprises a further sensor, which delivers an output signal that reflects the activity, metabolic demand, and/or ventricular potential, or which is a renal function sensor, pulmonary function sensor, cardiac function sensor, electrolyte sensor, neurosensor/vagus nerve/sympathetic nervous system, adverse event sensor, worsening heart failure sensor, sleep apnoea sensor, ischemia sensor or infarct sensor, and is connected to the control unit, wherein the control unit is configured to evaluate the output signal of said sensor and to use this to control the contractility modulation.

## Revendications

1. Stimulateur cardiaque
- avec au moins une unité de stimulation qui est reliée, ou est à relier, avec une ou plusieurs électrodes de stimulation, et conçue pour générer des impulsions de stimulation suite à un signal de départ correspondant pour la délivrance par au moins une électrode de stimulation,
- avec une unité de détection de rythme cardiaque qui est conçue pour détecter au moins un rythme cardiaque atrial et
- avec une unité de commande qui est reliée avec l'unité de stimulation et l'unité de détection de rythme cardiaque et est conçue pour commander une délivrance d'impulsions de stimulation par l'unité de stimulation de telle sorte que le stimulateur cardiaque peut délivrer des impulsions de stimulation au-delà d'un seuil pour l'excitation d'une contraction d'un ventricule et des impulsions de stimulations en deçà d'un seuil pour une thérapie de modulation de la contraction cardiaque,
où l'unité de commande est conçue pour, en réaction à la détection d'une arythmie atriale par l'unité de détection de rythme cardiaque, démarrer l'unité de stimulation de telle manière que celle-ci puisse émettre des impulsions de stimulation en deçà d'un seuil pour une thérapie de modulation de contraction cardiaque,
**caractérisé en ce**
**que** l'unité de commande est en outre conçue pour, en réaction suite à la détection d'un rythme sinusal par l'unité de détection de rythme cardiaque, démarrer l'unité de stimulation de telle sorte que celle-ci puisse délivrer des impulsions de stimulation au-delà d'un seuil pour l'excitation d'une contraction d'un ventricule, mais pas d'impulsions de stimulation en deçà d'un seuil pour une thérapie de modulation de contraction cardiaque.

2. Stimulateur cardiaque selon la revendication 1, **caractérisé en ce que** l'unité de détection de rythme cardiaque comprend une unité d'évaluation de rythme atrial qui est reliée, ou est à relier, avec une électrode atriale configurée pour la pose dans l'atrium droit d'un coeur.

3. Stimulateur cardiaque selon les revendications 1 ou 2, **caractérisé en ce que** l'unité de détection de rythme cardiaque, et éventuellement l'unité d'évaluation de rythme atrial associée à celle-ci, sont conçues pour évaluer la fréquence et la stabilité d'un rythme ventriculaire afin de diagnostiquer une fibrillation auriculaire.

4. Stimulateur cardiaque selon l'une des revendications 1 à 3, **caractérisé en ce que** le stimulateur cardiaque est un cardioverteur/défibrillateur implantable (ICD) et/ou un stimulateur cardiaque biventriculaire (CRT-D) et/ou un neurostimulateur.

5. Stimulateur cardiaque selon la revendication 4, **caractérisé en ce que** le stimulateur cardiaque est également simultanément un cardioverteur implantable pour la conversion électrique de fibrillations auriculaires, où l'unité de commande est configurée de telle sorte qu'une phase définie d'une thérapie CCM précède toujours une tentative de cardioversion de la défibrillation auriculaire pour l'amélioration de la réussite de la cardioversion.

6. Stimulateur cardiaque selon l'une des revendications 1 à 3, **caractérisé en ce que** le stimulateur cardiaque présente en outre un autre capteur qui donne un signal de sortie reflétant l'activité, le besoin métabolique et/ou le potentiel ventriculaire, ou un capteur de fonction rénale, un capteur de fonction pulmonaire, un capteur de fonction cardiaque, un capteur d'électrolyte, un neurocapteur /pneumogastrique / sympathique, un capteur d'évènement indésirable, un capteur d'une insuffisance cardiaque s'aggravant, d'un capteur d'apnée du sommeil, un capteur d'ischémie ou un capteur d'infarctus et qui est relié avec l'unité de commande, où l'unité de commande est conçue pour évaluer le signal d'émission de ce capteur et de l'utiliser pour la commande d'une modulation de contractilité.
